# EUROPÄISCHE PATENTSCHRIFT

(11) **EP 1 605 976 B1**
(45) Veröffentlichungstag und Bekanntmachung des Hinweises auf die Patenterteilung: **24.02.2010**
(21) Anmeldenummer: 04717614.4
(22) Anmeldetag: 05.03.2004
(51) Int. Cl.: A61K 31/4406, A61K 31/18, A61P 9/06

(54) **KOMBINATION VON PHENYLCARBONSÄUREAMIDEN MIT BLOCKERN DES IKR-KANALS UND DEREN VERWENDUNG ZUR BEHANDLUNG VON VORHOFARRHYTHMIEN**
PHENYLCARBOXYL ACID AMIDES AND IK r CHANNEL INHIBITORS COMBINATION AND THE USE THEREOF FOR TREATING ATRIAL ARRHYTHMIA
ASSOCIATION D'AMIDES DE L'ACIDE PHENYLCARBOXYLIQUE ET D'INHIBITEURS DU CANAL IKr ET UTILISATION DE CETTE ASSOCIATION POUR TRAITER DES ARYTHMIES ATRIALES

(30) Priorität: 18.03.2003 DE 10312061
(43) Veröffentlichungstag der Anmeldung: 21.12.2005
(73) Patentinhaber: Sanofi-Aventis Deutschland GmbH, 65929 Frankfurt am Main (DE)
(72) Erfinder: BRENDEL, Joachim, 61118 Bad Vilbel (DE); WIRTH, Klaus, 65830 Kriftel (DE); GOEGELEIN, Heinz, 60528 Frankfurt (DE); ALLESSIE, Maurits, Department of Physiology, NL-6200 MD Maastricht (NL); BLAAUW, Yuri, Department of Physiology, NL-6200 MD Maastricht (NL)
(86) Internationale Anmeldenummer: PCT/EP2004/002246
(87) Internationale Veröffentlichungsnummer: WO 2004/082716

(56) Entgegenhaltungen:
- WO-A-01/25189
- WO-A-02/088073
- WO-A-02/100825
- COURTEMANCHE MARC ET AL: "Ionic targets for drug therapy and atrial fibrillation-induced electrical remodeling: Insights from a mathematical model" CARDIOVASCULAR RESEARCH, Bd. 42, Nr. 2, Mai 1999 (1999-05), Seiten 477-489, XP002292320 ISSN: 0008-6363
- BRENDEL J ET AL: "Blockers of the Kv1.5 channel for the treatment of atrial arrhythmias" EXPERT OPINION ON THERAPEUTIC PATENTS 01 NOV 2002 UNITED KINGDOM, Bd. 12, Nr. 11, 1. November 2002 (2002-11-01), Seiten 1589-1598, XP002292321 ISSN: 1354-3776
- RODEN D M: "Current status of class III antiarrhythmic drug therapy." THE AMERICAN JOURNAL OF CARDIOLOGY. 26 AUG 1993, Bd. 72, Nr. 6, 26. August 1993 (1993-08-26), Seiten 44B-49B, XP009033903 ISSN: 0002-9149
- PEUKERT STEFAN ET AL: "Identification, synthesis, and activity of novel blockers of the voltage-gated potassium channel Kv1.5." JOURNAL OF MEDICINAL CHEMISTRY, Bd. 46, Nr. 4, 13. Februar 2003 (2003-02-13), Seiten 486-498, XP002292322 ISSN: 0022-2623
- BLAAUW YURI ET AL: "Synergistic class III action of blockade of Ikur/Ito (AVE 0118) and IKr (dofetilide/ibutilide) in electrically remodeled atria of the goat." CIRCULATION, Bd. 108, Nr. 17 Supplement, 28. Oktober 2003 (2003-10-28), Seiten IV-84, XP009033919 & AMERICAN HEART ASSOCIATION SCIENTIFIC SESSIONS 2003; ORLANDO, FL, USA; NOVEMBER 09-12, 2003 ISSN: 0009-7322

## Beschreibung

Die Erfindung betrifft die Kombination aus einem oder mehreren IKᵣ-Kanal-Bfockern, ausgewählt aus der Gruppe Dofetilide und Ibutilide, und aus einem Kv1.5-Blocker-der Formel Ib 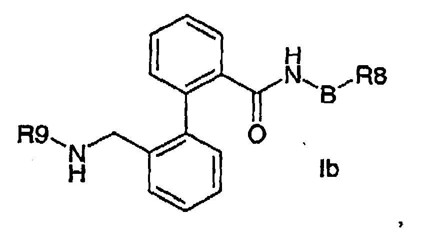 die 2'-{[2-(4-Methoxy-phenyl)-acetylamino]-methyl}-biphenyl-2-carbonsäure-(2-pyridin-3-yl-ethyl)-amid ist, und/oder pharmazeutisch verträglichen Salzen davon und die Verwendung der Kombination zur Behandlung von Vorhofarrhythmien.

Vorhof-Flimmem (AF) und Vorhof-Flattern sind die häufigsten anhaltenden Herzarrhythmien. Das Auftreten erhöht sich mit zunehmenden Alter und führt häufig zu fatalen Folgeerscheinungen, wie zum Beispiel Gehirnschlag. AF betrifft ca. 1 Millionen Amerikaner jährlich und führt zu mehr als 80.000 Schlaganfällen jedes Jahr in den USA. Die zur Zeit gebräuchlichen Antiarrhythmika der Klasse I und III reduzieren die Wiederauftrittsrate von AF, finden aber wegen ihrer potentiellen proarrhythmischen Nebenwirkungen nur eingeschränkte Anwendung. Deshalb besteht eine hohe medizinische Notwendigkeit für die Entwicklung besserer Medikamente zur Behandlung atrialer Arrhythmien (S. Nattel, Am. Heart J. 130, 1995, 1094 - 1106; "Newer developments in the management of atrial fibrillation").

Es wurde gezeigt, dass den meisten supraventrikulären Arrhythmien sogenannte "Reentry" Erregungswellen unterliegen. Solche Reentries treten dann auf, wenn das Herzgewebe eine langsame Leitfähigkeit und gleichzeitig sehr kurze Refraktärperioden besitzt. Das Erhöhen der myokardialen Refraktärzeit durch Verlängerung des Aktionspotentials ist ein anerkannter Mechanismus, um Arrhythmien zu beenden bzw. deren Entstehen zu verhindern (T. J. Colatsky et al, Drug Dev. Res. 19, 1990, 129 - 140; "Potassium channels as targets for antiarrhythmic drug action"). Die Länge des Aktionspotentials wird im wesentlichen bestimmt durch das Ausmaß repolarisierender K⁺-Ströme, die über verschiedene K⁺-Kanäle aus der Zelle herausfließen. Eine besonders große Bedeutung wird hierbei dem sogenannten "delayed rectifier" IK zugeschrieben, der aus 3 verschiedenen Komponenten besteht: IKᵣ, IK_{S} und IKᵤᵣ.

Die meisten bekannten Klasse III- Antiarrhythmika (zum Beispiel Dofetilide, Ibutilide, Almokalant, d-Sotalol) blockieren überwiegend oder ausschließlich den schnell aktivierenden Kaliumkanal IKᵣ, der sich sowohl in Zellen des menschlichen Ventrikel als auch im Vorhof nachweisen lässt. Es hat sich jedoch gezeigt, dass diese Verbindungen bei geringen oder normalen Herzfrequenzen ein erhöhtes proarrhythmisches Risiko aufweisen, wobei insbesondere Arrhythmien, die als "Torsades de pointes" bezeichnet werden, beobachtet wurden (D. M. Roden, Am. J. Cardiol. 72, 1993, 44B - 49B; "Current status of class III antiarrhythmic drug therapy"). Neben diesem hohen, zum Teil tödlichen Risiko bei niedriger Frequenz, wurde für die IKᵣ-Blocker ein Nachlassen der Wirksamkeit unter den Bedingungen von Tachykardie, in der die Wirkung gerade benötigt wird, festgestellt ("negative use-dependerice").

Die "besonders schnell" aktivierende und sehr langsam inaktivierende Komponente des delayed Rectifier IKᵤᵣ (=ultra-rapidly activating delayed rectifier), die dem Kr1.5-Kanal entspricht, spielt eine besonders große Rolle für die Repolarisationsdauer im menschlichen Vorhof. Eine Inhibierung des IKᵤᵣ-Kaliumauswärtsstroms stellt somit im Vergleich zur Inhibierung von IKᵣ bzw. IKₛ eine besonders effektive Methode zur Verlängerung des atrialen Aktionspotentials und damit zur Beendigung bzw. Verhinderung von atrialen Arrhythmien dar.
Im Gegensatz zu IKᵣ und IKₛ, die auch im menschlichen Ventrikel vorkommen, spielt der IKᵤᵣ zwar eine bedeutende Rolle im menschlichen Vorhof, jedoch nicht im Ventrikel. Aus diesem Grunde ist bei Inhibierung des IKᵤᵣ-Stroms im Gegensatz zur Blockade von IKᵣ oder IKₛ das Risiko einer proarrhythmischen Wirkung auf den Ventrikel von vornherein ausgeschlossen. (Z. Wang et al, Circ. Res. 73, 1993, 1061-1076: "Sustained Depolarisation-Induced Outward Current in Human Atrial Myocytes"; G.-R. Li et al, Circ. Res. 78, 1996, 689 - 696: "Evidence for Two Components of Delayed Rectifier K+-Current in Human Ventricular Myocytes"; G. J. Amos et al, J. Physiol. 491, 1996, 31 - 50: "Differences between outward currents of human atrial and subepicardial ventricular myocytes").

Antiarrhythmika, die über eine selektive Blockade des IKᵤᵣ₋Stroms bzw. Kv1.5-Kanals wirken, sind auf dem Markt bisher nicht verfügbar. Viele Patentanmeldungen beschreiben jedoch Verbindungen, die aufgrund ihrer blockierenden Wirkung auf den Kv1.5-Kanal als vorhofselektive Antiarrhythmika wirken. Zum Beispiel beschreibt die Patentanmeldung WO 0125189 unter anderem Biphenylcarbonamide als Kv1.5 Blocker. Die Anmeldungen WO 02088073 und WO 02100825 beschreiben Anthranilsäureamide als Kv1.5 Blocker zur Behandlung von Arrhythmien.

Es wurde nun überraschend gefunden, dass die antiarrhythmische Wirkung auf den kranken Herzvorhof_des Kv1.5-Blockers 2'-{[2-(4-Methoxy-phenyl)-acetylamino]-methyl}-biphenyl-2-carbonsäure-(2-pyridin-3-yl-ethyl)-amid-durch gleichzeitige Gabe eines IKᵣ-Kanal-Blockers, ausgewählt aus der Gruppe Dofetilide und Ibutilide, synergistisch verstärkt werden kann.

Die gleichzeitige Gabe eines Kv1.5-Blockers mit einem IKᵣ-Kanal-Blocker wie zum Beispiel Ibutilide führt am normalen, d.h. gesunden, Vorhof lediglich zu einer Addition der Einzeleffekte. Im flimmernden Herzvorhof kommt es jedoch im Verlaufe von 1 bis 4 Tagen zu einer Veränderung der lonenkanalzusammensetzung (Elektrisches Remodelling). Unter diesen Bedingungen, die die,Situation am flimmernden Patienten besser wiederspiegeln als der gesunde Vorhof, ist die Wirkung des IKᵣ-Blockers allein deutlich reduziert. Es wurde überraschend gefunden, dass IKᵣ-Kanalblocker, die alleine im remodellierten Zustand eine deutlich reduzierte Wirkung auf die Refraktärzeit zeigen, in Kombination mit dem IKᵤᵣ-Blocker wieder voll Refraktärzeitwirksam werden. Das heißt, die Blockade des IKᵤᵣ stellt offenbar die im chronischen Flimmern verloren gegangene Wirkung der IKᵣ-Blocker wieder her. Eine Kombination von einem IKᵣ-Kanalblocker, ausgewählt aus der Gruppe Dofetilide und Ibutilide, mit dem Kv1.5-Blocker 2'-{[2-(4-Methoxy-phenyl)-acetylamino]-mathyl}-biphenyl-2-carbonsäure-(2-pyridin-3-yl-ethyl)-amid führt deshalb zu einer deutlich überadditiven Verstärkung der Wirkung auf die atriale effektive Refraktärzeit (AERP). Da die Verlängerung der AERP einen anerkannten Surrogatparameter für die antiarrhythmische Wirkung einer Substanz darstellt, ist hiermit auch die synergistische antiarrhythmische Wirkung der Kombination belegt.

Die überraschend starke Wirkung der Kombination auf die Verlängerung der effektiven Refraktärzeit führt auch zu einer wesentlich höheren Erfolgsrate bei der Kardioversion. Ein persistierendes Flimmern, also ein Flimmern, das schon länger als einen Monat auftritt, kann durch kein bekanntes Medikament beendet werden. Die IKᵣ-Blocker wie zum Beispiel lbutilide oder Dofetilide allein führen in diesem Modell lediglich zu einer leichten Verlängerung der Zykluslänge des atrialen Flimmerns (Atrial Fibrillation Cycle Length = AFCL), jedoch zu keiner Terminierung des Flimmems. Mit dem Kv1.5-Blocker des Beispiels 1 allein gelang in der Dosierung von zum Beispiel 10 mg/kg eine Kardioversion. Die Kombination von der Verbindung des Beispiels 1 und Ibutilide oder Dofetilide, zum Beispiel, hingegen führte jedoch überraschenderweise bereits bei Verabreichung des Kv1.5-Biockers in Dosen von zum Beispiel 0,3 bis 3 mg/kg zusammen mit sehr niedrigen Dosen (zum Beispiel 10 µg/kg) des IKᵣ-Kanalblockers zu einer Terminierung des Flimmerns. Die Kombination der beiden Wirkprinzipien ermöglicht somit eine deutliche Reduzierung der einzusetzenden Wirkstoffdosen und damit ein erheblich verbessertes Verhältnis aus gewünschten Wirkungen und unerwünschten Nebenwirkungen.

Die hier beschriebenen Kombinationen von Kv1.5- und IKᵣ-Blockem können deshalb verwendet werden als hochwirksame Antiarrhythmika mit besonders vorteilhaftem Sicherheitsprofil. Insbesondere eignen sich die Verbindungen zur Behandlung supraventrikulärer Arrhythmien, zum Beispiel Vorhof-Flimmern oder Vorhof-Flattern. Die Kombinationen können eingesetzt werden zur Terminierung von bestehendem Vorhof-Flimmern oder -Flattern zur Wiedererlangung des Sinus-Rhythmus (Kardioversion). Durch die deutlich verstärkte Wirkung der Kombination können auch Patienten mit persistierendem Flimmern kardiovertiert werden, die bisher einer medikamentösen Behandlung nicht zugänglich waren. Darüber hinaus reduzieren die Kombinationen die Anfälligkeit zur Entstehung neuer Flimmer-Ereignisse (Erhalt des Sinus-Rhythmus, Prophylaxe).

Die Erfindung betrifft die Kombination aus einem oder mehreren IKᵣ-Kanalblockern, ausgewählt aus der Gruppe Dofetilide und Ibutilide, und 2'-{[2-(4-Methoxy-phenyl)-acetylamino]-methyl}-biphenyl-2-carbonsäure-(2-pyridin-3-yl-ethyl)-amid und/oder ihren physiologisch verträglichen Salzen.

Weiterhin betrifft die Erfindung die Verwendung eines oder mehrerer IKᵣ-Kanalblocker, ausgewählt aus der Gruppe Dofetilide und Ibutilide, zusammen mit 2'-{[2-(4-Methoxyphenyl)-acetytamino]-methyl}-biphenyl-2-carbonsäure-(2-pyridin-3-yl-ethyl)-amid und/oder ihren physiologisch verträglichen Salzen.

Bevorzugt ist die Verwendung eines oder mehrerer IKᵣ-Kanalblocker zusammen mit 2'-{[2-(4-Methoxy-phenyl)-acetylamino]-methyl}-biphenyl-2-carbonsäure-(2-pyridin-3-yl-ethyl)-amid und/oder eines physiologisch verträglichen Salzes davon zur Herstellung eines Medikamentes zur Therapie oder Prophylaxe von atrialer Fibrillation oder atrialem Flattern, wobei die IKᵣ-Blocker ausgewählt sind aus der Gruppe Dofetilide und Ibutilide.

Enthalten die Verbindungen der Formel Ib eine oder mehrere saure oder basische Gruppen bzw. einen oder mehrere basische Heterocyclen, so gehören auch die entsprechenden physiologisch oder toxikologisch verträglichen Salze zur Erfindung, insbesondere die pharmazeutisch verwendbaren Salze. Verbindungen der Formel Ib, die einen Pyridin- oder Chinolinsubstituent enthalten, können auch in Form ihrer physiologisch verträglichen Säureadditionssalze mit anorganischen oder organischen Säuren verwendet werden, beispielsweise als Hydrochloride, Phosphate, Sulfate, Methansulfonate, Acetate, Lactate, Maleinate, Fumarate, Malate, Gluconate usw.

Entsprechend können die IKᵣ-Kanalblocker in Form ihrer physiologisch verträglichen Salze eingesetzt werden.

Die Verbindungen der Formel I können bei entsprechender Substitution in stereoisomeren Formen vorliegen. Enthalten die Verbindungen der Formel Ib ein oder mehrere Asymmetriezentren, so können diese unabhängig voneinander die S-Konfiguration oder die R-Konfiguration aufweisen. Zur Erfindung gehören alle möglichen Stereoisomeren, zum Beispiel Enantiomere oder Diastereomere, und Mischungen von zwei oder mehr stereoisomeren Formen, zum Beispiel Enantiomeren und/oder Diastereomeren, in beliebigen Verhältnissen. Enantiomere zum Beispiel gehören also in enantiomerenreiner Form, sowohl als links- als auch als rechtsdrehende Antipoden, und auch in Form von Mischungen der beiden Enantiomeren in unterschiedlichen Verhältnissen oder in Form von Racematen zu der Erfindung. Die Herstellung von einzelnen Stereoisomeren kann gewünschtenfalls durch Auftrennung eines Gemisches nach üblichen Methoden oder zum Beispiel durch Verwendung isomerenreiner Synthesebausteine erfolgen.
Als geeignete IKᵣ-Kanalblocker können zum Beispiel die in Tabelle 1 aufgeführten Substanzen verwendet werden.

**Tabelle 1: Namen und Strukturformeln von exemplarischen IKᵣ-Kanalbtockern**

| Name | Struktur |
|---|---|
| Dofetilide | 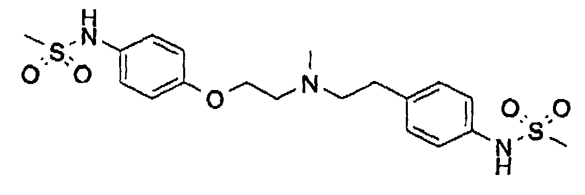 |
| Ibutilide | 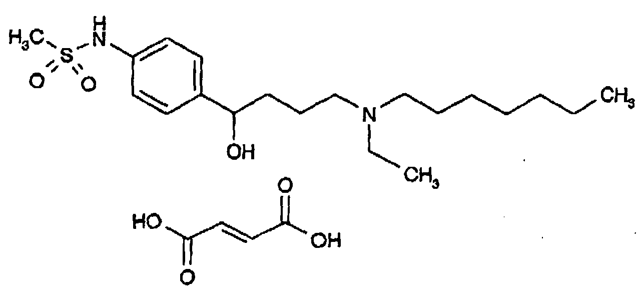 |

Die erfindungsgemäß verwendete Verbindung 2'-{[2-(4-Methoxy-phenyl)-acetylamino]-methyl}-biphenyl-2-Carbonsäure-(2-pyridin-3-yl-ethyl)-amid und/oder ihre physiologisch verträglichen Salze können somit zusammen mit einem oder mehreren IKᵣ-Kanalblocker, ausgewählt aus der Gruppe Dofetilide und Ibutilide, am Tier, bevorzugt am Säugetier, und insbesondere am Menschen in vorteilhafter Weise als Arzneimittel verwendet werden, insbesondere zur Behandlung von atrialen Arrhythmien.

Die Kombination der beiden Wirkstoffe kann derart erfolgen, dass 2'-{[2-(4-Methoxyphenyl)-acetylamino]-methyl}-biphenyl-2-carbonsäure-(2-pyridin-3-yl-ethyl)-amid und ein oder mehrere IKᵣ-Kanalblocker, ausgewählt aus der Gruppe Dofetilide und Ibutilide, zusammen in einem Medikament verabreicht werden oder dass ein Medikament, welches 2'-{[2-(4-Methoxy-phenyl)-acetylamino]-methyl}-biphenyl-2-carbonsäure-(2-pyridin-3-yl-ethyl)-amid enthält und ein separates Medikament, das einen oder mehrere IKᵣ-Blocker, ausgewählt aus der Gruppe Dofetilide und Ibutilide, enthält, gleichzeitig oder nacheinander in jeder Reihenfolge verabreicht werden. Eine Verabreichung nacheinander schließt auch eine Kombination ein, bei der die einzelnen Medikamente zu unterschiedlichen Zeiten und auf unterschiedlichen Wegen verabreicht werden, um einen besseren Effekt zu erzielen. Es kann aber auch zweckmäßig sein, zunächst eine geeignete Dosis des einen Medikamentes zu verabreichen und anschließend das andere Medikament solange zu verabreichen, zum Beispiel durch Infusion, bis der gewünschte Kombinationseffekt, zum Beispiel die Kardioversion in den Sinusryhthmus, eingetreten ist. Je nach den Gegebenheiten des Einzelfalles kann es günstiger sein, 2'-{[2-(4-Methoxy-phenyl)-acetylamino]-methyl}-biphenyl-2-carbonsäure-(2-pyridin-3-yl-ethyl)-amid und einen oder mehrere IKᵣ-Kanalblocker, ausgewählt aus der Gruppe Dofetilide und Ibutilide, in Form eines pharmazeutischen Kombinationspräparats zu verabreichen, in dem die beiden Wirkstoffe in einem festen Mengenverhältnis vorliegen, oder sie in Form von separaten pharmazeutischen Einzelpräparaten zu verabreichen. Im letzteren Falle, in dem das Mengenverhältnis der beiden Wirkstoffe variiert werden kann, können sich die Einzelpräparate in geeigneter Primärverpackung und gegebenenfalls zusammen mit einer auf die erfindungsgemäße Verwendung verweisenden Gebrauchsanweisung in einer gemeinsamen Verpackung befinden, oder die Einzelpräparate können sich gegebenenfalls jeweils zusammen mit auf die erfindungsgemäße Verwendung verweisenden Gebrauchsanweisungen in getrennten Verpackungen befinden. Alle derartigen Erzeugnisse und Herrichtungsarten werden von der vorliegenden Erfindung umfasst. Gegenstand der Erfindung ist also beispielsweise ein Erzeugnis enthaltend eine Kombination aus einem oder mehreren IKᵣ-Kanalblockern, ausgewählt aus der Gruppe Dofetilide und Ibutilide, und aus 2'-{[2-(4-Methoxy-phenyl)-acetylamino]-methyl}-biphenyl-2-carbonsäure-(2-pyrldin-3-yl-ethyl)-amid und/oder physiologisch verträglicher Salze davon zur gleichzeitigen, getrennten oder zeitlich abgestuften Anwendung zur Therapie oder Prophylaxe von atrialer Fibrillation oder atrialern Flattern.

Das Gewichtsverhältnis von den Wirkstoffen 2'-{[2-(4-Methoxy-phenyl)-acetylamino]-methyl}-biphenyl-2-carbonsäure-(2-pyridin-3-yl-ethyl)-amid zu dem oder den IKᵣ-Kanalblockern, ausgewählt aus der Gruppe Dofetilide und Ibutlilde, in den erfindungsgemäßen Kombinationen liegt üblicherweise in einem Bereich von 10000:1 1 bis 1 : 1, bevorzugt zwischen 100 : 1 und 5 : 1.

Gegenstand der vorliegenden Erfindung ist auch die Verwendung 2'-{[2-(4-Methoxyphenyl)-acetylamino]-methyl}-biphenyl-2-carbonsäure-(2-pyridin-3-yl-ethyl)-amid und/oder eines physiologisch verträgliche Salzes davon und eines oder mehrer IKᵣ-Blocker, ausgewählt aus der Gruppe Dofetilide und Ibutilide, zur Herstellung pharmazeutischer Zubereitungen, die 2'-{[2-(4-Methoxy-phenyl)-aoetylamino]-methyl}-biphenyl-2-carbonsäure-(2-pyridin-3-yl-ethyl)-amid und eine oder mehrere der IKᵣ-Kanal-Blocker, ausgewählt aus der Gruppe Dofetilide und Ibutilide, als wirksame Komponenten neben üblichen, pharmazeutisch einwandfreien Trägerstoffen enthalten, und deren Verwendung als Medikament zur Behandlung von beispielsweise atrialen Arrhythmien.

Weiterhin sind Gegenstand der vorliegenden Erfindung pharmazeutische Zubereitungen (Kombinationspräparat), die als aktiven Bestandteil eine wirksame Dosis von 2'-{[2-(4-Methoxy-phenyl)-acetylamino]-methyl}-biphenyl-2-carbonsäure-(2-pyridin-3-yl-ethyl)-amid und/oder eines physiologisch verträgliches Salzes davon und mindestens eines IKᵣ-Kanalblockers, ausgewählt aus der Gruppe Dofetilide und Ibutilide, und/oder eines physiologisch verträglichen Salzes davon neben üblichen, pharmazeutisch einwandfreien Träger- und Hilfsstoffen enthalten und gegebenenfalls noch einem oder mehreren anderen pharmakologischen Wirkstoffen. Die pharmazeutischen Zubereitungen enthalten normalerweise 0,1 bis 90 Gewichtsprozent der Verbindunge 2'-{[2-(4-Methoxy-phenyl)-acetylamino]-methyl}-biphenyl-2-carbonsäure-(2-pyridin-3-yl-ethyl)-amid und/oder ihrer physiologisch verträglichen Salze und der IKᵣ-Kanalblocker, ausgewählt aus der Gruppe Dofetilide und Ibutilide, und/oder ihrer physiologisch verträglichen Salze.

Die Herstellung der pharmazeutischen Zubereitungen kann in an sich bekannter Weise erfolgen. Dazu werden die Wirkstoffe und/oder ihre physiologisch verträglichen Salze zusammen mit einem oder mehreren festen oder flüssigen galenischen Trägerstoffen und/oder Hilfsstoffen in eine geeignete Darreichungsform bzw. Dosierungsform gebracht, die dann als Arzneimittel in der Humanmedizin oder Veterinärmedizin verwendet werden kann. Entsprechendes gilt auch für pharmazeutische Zubereitungen, die die beiden Wirkstoffe Kv1.5-Blocker 2'-{[2-(4-Methoxy-phenyl)-acetylamino]-methyl}-biphenyl-2-carbonsäure-(2-pyridin-3-yl-ethyl)-amid und IKᵣ-Blocker, ausgewählt aus der Gruppe Dofetilide und Ibutilide, und/oder deren pharmazeutisch verträglichen Salze separat enthalten.

Arzneimittel, die erfindungsgemäße Kombinationen aus 2'-{[2-(4-Methoxy-phenyl)-acetylamino]-methyl}-biphenyl-2-carbonsäure-(2-pyridin-3-yl-ethyl)-amid und/oder ihre physiologisch verträglichen Salze und aus einem oder mehreren IKᵣ-Blockern, ausgewählt aus der Gruppe Dofetilide und Ibutilide, und/oder ihre physiologisch verträglichen Salze enthalten bzw. die in Kombination eingesetzten Einzelkomponenten, können zum Beispiel oral, parenteral, intravenös, rektal, durch Inhalation oder topisch appliziert werden, wobei die bevorzugte Applikation vom Einzelfall abhängig ist.

Beansprucht werden insbesondere Kombinationspräparate aus 2'-{[2-(4-Methoxyphenyl)-acetylamino]-methyl}-biphenyl-2-carbonsäure-(2-pyridin-3-yl-ethyl)-amid und/oder ihre physiologisch verträglichen Salze und einem oder mehreren IKᵣ-Blockern, ausgewählt aus der Gruppe Dofetilide und Ibutilide, und/oder ihre physiologisch verträglichen Salze zur Behandlung von atrialen Arrhythmien wie Vorhof-Flimmem und Vorhof-Flattem.

Welche Hilfsstoffe für die gewünschte Arzneimittelformulierung geeignet sind, ist dem Fachmann auf Grund seines Fachwissens geläufig. Neben Lösemitteln, Gelbildnern, Suppositoriengrundlagen, Tablettenhilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Mittel zur Erzielung eines Depoteffekts, Puffersubstanzen oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel, vermischt und durch die üblichen Methoden in die geeigneten Darreichungsformen gebracht, wie Tabletten, Dragees, Steckkapseln, wässrige, alkoholische oder ölige Lösungen. Als inerte Träger können zum Beispiel Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran. Als Lösungsmittel für wässrige oder alkoholische Lösungen kommen zum Beispiel Wasser, Ethanol oder Zuckerlösungen oder Gemische davon, in Betracht. Weitere Hilfsstoffe, auch für andere Applikationsformen, sind zum Beispiel Polyethylenglykole und Polypropylenglykole.

Zur subkutanen, intramuskulären oder intravenösen Applikation werden die aktiven Verbindungen, gewünschtenfalls mit den dafür üblichen Substanzen wie Lösungsvermittlern, Emulgatoren oder weiteren Hilfsstoffen, in Lösung, Suspension oder Emulsion gebracht. Als Lösungsmittel kommen zum Beispiel Wasser, physiologische Kochsalzlösung oder Alkohole, zum Beispiel Ethanol, Propanol, Glycerin, in Betracht, daneben auch Zuckerlösungen wie Glucose- oder Mannitlösungen, oder auch Mischungen aus den verschiedenen genannten Lösungsmitteln.

Als pharmazeutische Formulierung für die Verabreichung in Form von Aerosolen oder Sprays sind geeignet zum Beispiel Lösungen, Suspensionen oder Emulsionen der Wirkstoffe oder ihrer physiologisch verträglichen Salze in einem pharmazeutisch unbedenklichen Lösungsmittel, wie insbesondere Ethanol oder Wasser, oder einem Gemisch solcher Lösungsmittel. Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gewichtsprozent.

Die Dosierung der erfindungsgemäß zu verabreichenden aktiven Verbindungen bzw. der physiologisch verträglichen Salze davon hängt vom Einzelfall ab und ist wie üblich für eine optimale Wirkung den Gegebenheiten des Einzelfalls anzupassen. So hängt sie natürlich ab von der Häufigkeit der Verabreichung und von der Wirkstärke und Wirkdauer der jeweils zur Therapie oder Prophylaxe eingesetzten Verbindungen, aber auch von Art und Stärke der zu behandelnden Krankheit sowie von Geschlecht, Alter, Gewicht und individueller Ansprechbarkeit des zu behandelnden Menschen oder Tieres und davon, ob akut oder chronisch therapiert wird oder Prophylaxe betrieben wird. Insbesondere bei der Behandlung akuter Fälle von Herzrhythmusstörungen, beispielsweise auf einer Intensivstation, kann auch eine parenterale Verabreichung durch Injektion oder Infusion, zum Beispiel durch eine intravenöse Dauerinfusion, vorteilhaft sein, wenn die Verbindungen am Tier, bevorzugt am Säugetier, und insbesondere am Menschen als Arzneimittel verwendet werden.

Die Dosierung des Kv1.5-Blockers 2'-{[2-(4-Methoxy-phenyl)-acetylamino]-methyl}-biphenyl-2-carbonsäure-(2-pyridin-3-yl-ethyl)-amid kann üblicherweise im Bereich von 1 mg bis 1 g pro Tag und pro Mensch (bei etwa 75 kg Körpergewicht) variieren, bevorzugt von 5 bis 750 mg pro Tag und Mensch. Bei dem IKᵣ-Blocker, ausgewählt aus der Gruppe Dofetillde und Ibutilide, kann die Dosis üblicherweise zwischen 1 µg und 10 mg pro Tag und Mensch variieren, bevorzugt zwischen 5 bis 500 µg pro Tag und Mensch. Es können aber auch höhere Dosen angebracht sein.

Bei der erfindungsgemäßen Kombinationsbehandlung können der Kv1.5-Blocker 2'-{[2-(4-Methoxy-phenyl)-acetylamino]-methyl}-biphenyl-2-carbonsäure-(2-pyridin-3-yl-ethyl)-amid und der oder die IKᵣ-Blocker, ausgewählt aus der Gruppe Dofetilide und Ibutilide, und/oder ihre physiologisch verträglichen Salze in geringeren Dosen verabreicht werden als bei Verabreichung nur eines der beiden Wirkstoffe

Bei der erfindungsgemäßen Kombinationsbehandlung kann die Tagesdosis der Wirkstoffe auf einmal verabreicht werden oder sie kann auf mehrere, zum Beispiel zwei, drei oder vier Verabreichungen aufgeteilt werden.

### Experimenteller Teil

### Liste der Abkürzungen

- DMAP: 4-Dimethylaminopyridin
- EDAC: N-Ethyl-N'-(3-dimethylaminopropyl)-carbodiimid Hydrochlorid
- HOBT: 1-Hydroxy-1H-benzotriazol
- RT: Raumtemperatur
- THF: Tetrahydrofuran

### Beispiel 1: 2'-{[2-(4-Methoxy-phenyl)-acetylamino]-methyl}-biphenyl-2-carbonsäure-(2-pyridin-3-yl-ethyl)-amid

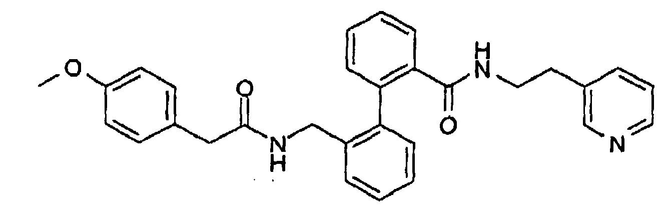

Zu einer Lösung von 37,8 g (0,11 mol) 2'-(tert-Butoxycarbonylamino-methyl)-biphenyl-2-carbonsäure (Brandmeier, V.; Sauer, W.H.B.; Feigel, M. ; Helv. Chim. Acta 1994, 77(1), 70-85) in 550 ml THF wurden 15,5 g (0,115 mol) HOBT und 21,9 g (0,115 mol) EDAC zugegeben und die Reaktionsmischung wurde 45 min bei Raumtemperatur gerührt. Anschließend wurden 14,0 g (0,115 mol) 3-(2-Aminoethyl)-pyridin zugegeben und es wurde über Nacht bei RT gerührt. Nach Zugabe von 400 ml Wasser und 500 ml Essigester und intensivem Rühren, wurden die Phasen getrennt. Die organische Phase wurde 1 mal mit 400 ml gesättigter Natriumchloridlösung und 2mal mit je 400 ml gesättigter Natriumhydrogencarbonatlösung gewaschen. Nach Trocknen über Magnesiumsulfat in Gegenwart von Aktivkohle wurde filtriert und am Rotationsverdampfer eingeengt.
Das erhaltene Zwischenprodukt (40,7 g) wurde in 600 ml Methylenchlorid gelöst und dann wurden 100 ml Trifluoressigsäure langsam zugetropft. Nach Rühren über Nacht wurde die Reaktionsmischung in Vakuum eingeengt. Der Rückstand wurde mit 250 ml Essigester versetzt und erneut eingeengt, um überschüssige Trifluoressigsäure herauszudestillieren. Zu dem erhaltenen Rohprodukt gelöst in 170 ml Methylenchlorid wurden 72,8 ml (530 mmol) Triethylamin zugetropft und 1 g DMAP hinzugefügt. Anschließend wurden bei 5 - 10°C 18,7 g (100 mmol) 4-Methoxyphenylessigsäurechlorid innerhalb von 30 min zugetropft, und der Ansatz wurde über Nacht bei Raumtemperatur gerührt. Nach Zugabe von 150 ml Wasser und intensivem Rühren wurden die Phasen getrennt und die organische Phase wurde 1 mal mit 100 ml Natriumchlorid-Lösung, 1mal mit 25 ml 1 M Salzsäure und 2mal mit je 100 ml gesättigter Natriumhydrogencarbonatlösung gewaschen. Nach Trocknen über Magnesiumsulfat und Aktivkohle, wurde im Vakuum eingeengt. Das erhaltene Öl wurde in Acetonitril heiß gelöst und langsam auskristallisieren gelassen.
Es wurden 21,5 g 2'-{[2-(4-Methoxy-phenyl)-acetylamino]-methyl}-biphenyl-2-carbonsäure-(2-pyridin-3-yl-ethyl)-amid, Schmelzpunkt 116°C, erhalten.

### Beispiel 2 : 2'-(Benzyloxycarbonylamino-methyl)-biphenyl-2-carbonsäure-2-(2-pyridyl)-ethylamid (not part of the invention)

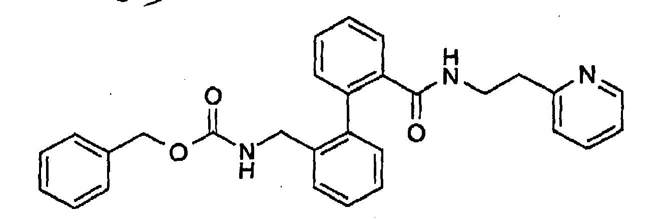

Die Verbindung wurde erhalten gemäß der in WO 0125189 angegebenen Synthesevorschrift.

### Beispiel 3:2'-{[2-(4-Methoxy-phenyl)-acetylamino]-methyl}-biphenyl-2-carbonsäure-2,4=difluor-benzylamid (not part of the invention)

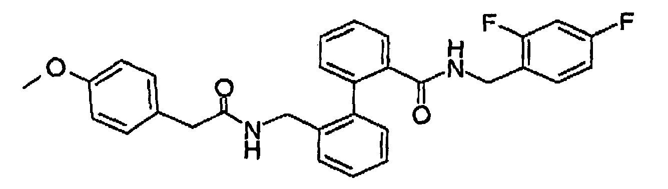

Die Verbindung wurde erhalten gemäß der in WO 0125189 angegebenen Synthesevorschrift.

### Beispiel 4: (S)-2'-(α-Methyl-benzyloxycarbonyl-aminomethyl)-biphenyl-2-carbonsäure-2-(2-pyridyl)-ethylamid (not part of the invention)

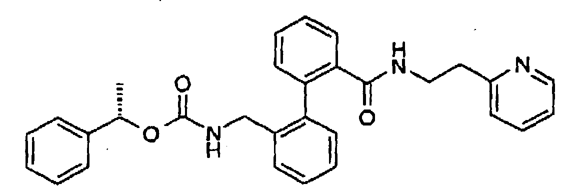

Die Verbindung wurde erhalten gemäß der in WO 0125189 angegebenen Synthesevorschrift.

### Beispiel 5: 2-(Butyl-1-sulfonylamino)-N-[1(R)-(6-methoxy-pyridin-3-yl)-propyl]-benzamid (not part of the invention)

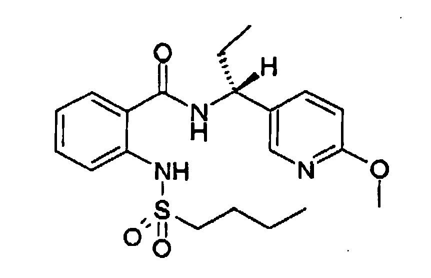

### a) 2-(Butyl-1-sulfonylamino)-benzoesäure

Zu einer Suspension von 20 g (146 mmol) 2-Aminobenzoesäure in 250 ml Wasser wurden 20 g (188 mmol) Natriumcarbonat zugefügt. Anschließend wurden 11,4 g (72,8 mmol) Butylsulfonylchlorid zugetropft und die Reaktionsmischung wurde 2 Tage bei Raumtemperatur gerührt. Es wurde mit konzentrierter Salzsäure angesäuert, 3 Stunden bei Raumtemperatur gerührt und das ausgefallene Produkt abgesaugt. Nach Trocknen im Vakuum erhielt man 9,6 g 2-(Butyl-1-sutfonylamino)-benzoesäure.

### b)1-(6-Methoxy-pyridin-3-yl)-propylamin

Zu einer Lösung von 10,2 ml Butyllithium (2,5 M Lösung in Hexan; 25,5 mmol) in 50 ml Diethylether wurden bei -70°C 3 ml (23,2 mmol) 5-Brom-2-methoxypyridin zugegeben. Nach 10 min wurden 1,4 ml (19.5 mmol) Propionitril zugegeben. Nach 2 Stunden bei -70°C wurde die Reaktionsmischung langsam auf Raumtemperatur kommen gelassen. Dann wurden 2,2 g Natriumsulfat Dekahydrat zugesetzt und 1 Stunde Rühren gelassen. Nach anschließender Zugabe von 5 g Magnesiumsulfat wurde nach kurzem Rühren von den Salzen abfiltriert und das Filtrat wurde eingeengt. Der Rückstand wurde in 70 ml Methanol gelöst und bei 0°C wurden 1,1 g (28 mmol) Natriumborhydrid zugegeben. Nach Rühren über Nacht, wurde die Reaktionsmischung mit konzentrierter Salzsäure auf pH 2 gestellt und am Rotationsverdampfer eingeengt. Der Rückstand wurde mit 10 ml Wasser versetzt, und einmal mit Diethylether extrahiert. Anschließend, wurde die wäßrige Phase mit Natriumhydrogencarbonat gesättigt, im Vakuum eingeengt und der Rückstand mit Essigsäureethylester extrahiert. Nach Trocknen und Einengen der Essigesterextrakte wurden 1,4 g razemisches 1-(6-Methoxy-pyridin-3-yl)-propylamin erhalten.

### c) 2-(Butyl-1-sulfonylamino)-N-[1(R)-(6-methoxy-pyndin-3-yl)-propyl]-benzamid

Zu einer Lösung von 8,0 g (31,1 mmol) 2-(Butyl-1-sulfonylamino)-benzoesäure in 250 ml Tetrahydrofuran wurden 4,4 g (32, 7 mmol) 1-Hydroxy-1H-benzotriazol und 6,3 g (32,7 mmol) N-Ethyl-N'-(3-dimethylaminopropyl)-carbodiimid Hydrochlorid zugefügt und die Reaktionsmischung wurde 90 min gerührt. Dann wurde eine Lösung von 5,4 g (32,7 mmol) razemisches 1-(6-Methoxy-pyridin-3-yl)-propylamin in 20 ml Tetrahydrofuran zugetropft und über Nacht gerührt. Die Reaktionsmischung wurde mit 250 ml Wasser versetzt und mit 300 ml Essigsäureethylester extrahiert. Die organische Phase wurde 5mal mit je 100 ml gesättigter Natriumhydrogencarbonatlösung extrahiert und dann über Magnesiumsulfat getrocknet. Man erhielt 9,0 g 2-(Butyl-1-sulfonylamino)-N-[1-(6-methoxy-pyridin-3-yl)-propyl]-benzamid.
Die Trennung der Enantiomere erfolgte durch präparative HPLC auf einer Chiralpak ADH Säule (250x4,6 mm); Eluent: Heptan / Ethanol / Methanol 10:1:1; Temperatur: 30°C; Flußrate: 1 ml /min. Als erstes wurden bei einer Retentionszeit von 5,9 min 4,0 g 2-(Butyl-1-sulfonylamino)-N-[1(R)-(6-methoxy-pyridin-3-yl)-propyl]-benzamid eluiert. Nach einer Mischfraktion wurden bei einer Retentionszeit von 7,2 min 3,0 g 2-(Butyl-1-sulfonylamino)-N-[1(S)-(6-methoxy-pyridin-3-yl)-propyl]-benzarnid erhalten.

2 g des 2-(Butyl-1-sulfonylamino)-N-[1(R)-(6-methoxy-pyridin-3-yl)-propyl]-benzamids wurden in 9 ml Isopropanol in der Hitze gelöst, dann wurden 8 ml warmes Wasser zugegeben und die Reaktionsmischung wurde über Nacht langsam abkühlen gelassen. Nach Absaugen bei 0°C wurden 1,5 g 2-(Butyl-1-sulfonylamino)-N-[1(R)-(6-methoxy-pyridin-3-yl)-propyo-benzamid als farblose nadelförmige Kristalle erhalten; Schmelzpunkt 97°C.

### Beispiel 6: 2-(Butyl-1-sulfonylamino)-N-(cyclopropyl-pyridin-3-yl-methyl)-5-methyl-benzamid (not part of the invention)

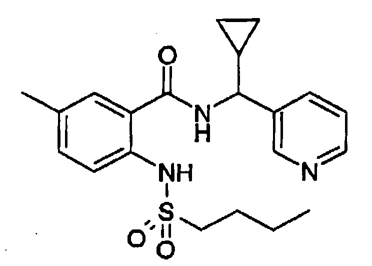

Die Verbindung wurde erhalten gemäß der in WO 02088073 angegebenen Synthesevorschrift.

### Beispiel 7: (S)-5-Fluor-2-(chinolin-8-sulfonylamino)- N-(1-phenyl-propyl)-benzamid (not part of the invention)

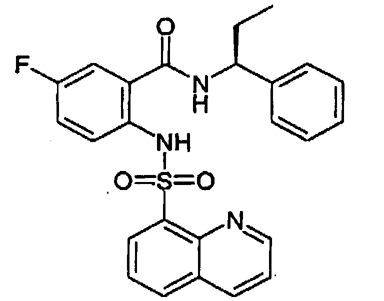

### a) 5-Fluor-2-(chinolin-8-sulfonylamino)-benzoesäure

Eine Reaktionsmischung aus 10,0 g (64 mmol) 5-Fluor-2-amino-benzoesäure, 16,3 g (193 mmol) Natriumhydrogencarbonat und 16,3 g 8-Chinolinsulfonylchlorid in 325 ml Wasser und 325 ml Essigester wurde über Nacht bei RT gerührt. Die wässerige Phase wurde abgetrennt und 1-mal mit 50 ml Essigester extrahiert. Anschließend wurde die wässerige Phase mit konz. Salzsäure sauer gestellt und 2 h gerührt. Der ausgefallene Niederschlag wurde abgesaugt, im Vakuum getrocknet und man erhielt 19,5 g 5-Fluor-2-(chinolin-8-sulfonylamino)-benzoesäure.

### b) 5-Fluor-2-(chinolin-8-sulfonylamino)- N-(1-phenyl-propyl)-benzamid

Aus 5,5 g (15,9 mmol) 5-Fluor-2-(Chinolin-8-sulfonylamino)-benzoesäure und 2,3 g (16,7 mmol) (S)-Phenylpropylamin wurden gemäß der Vorschrift in WO02100825 5,7 g der Titelverbindung erhalten.
Fp.: 163°C

### Beispiel 8: (S)-5-Fluor-2-(chinotin-8-sulfonylamino)- N-(1-phenyl-propyl)-benzamid-Natriumsalz (not part of the invention)

Zu einer Lösung von 5 g der Verbindung des Beispiels 7 in 120 ml Essigester wurden 2 ml einer 30-prozentigen Natriummethanolatlösung gegeben. Das ausgefallene Natriumsalz wurde abgesaugt und aus 25 ml Ethanol umkristallisiert und man erhielt 3,3 g der Titelverbindung.

### Pharmakologische Untersuchungen

### Bestimmung der Aktivität auf den Kv1.5-Kanal

Kv1.5-Kanäle aus dem Menschen wurden in Xenopus Oozyten expremiert. Hierfür wurden zuerst Oozyten aus Xenopus laevis isoliert und defollikuliert. Anschließend wurde in diese Oozyten in vitro synthetisierte Kv1.5 kodierende RNA injiziert. Nach 1 - 7 Tagen Kv1.5-Proteinexpression wurden an den Oozyten mit der Zwei-Mikroelektroden Voltage-Clamp Technik Kv1.5-Ströme gemessen. Die K1.5-kanäle wurden hierbei in der Regel mit 500 ms dauernden Spannungssprüngen auf 0 mV und 40 mV aktiviert. Das Bad wurde mit einer Lösung der nachfolgenden Zusammensetzung durchspült. NaCl 96 mM, KCl 2 mM, CaCl₂ 1,8 mM, MgCl₂ 1 mM, HEPES 5 mM (titriert mit NaOH auf pH 7,4). Diese Experimente wurden bei Raumtemperatur durchgeführt. Zur Datenerhebung und Analyse wurden eingesetzt: Geneclamp Verstärker (Axon Instruments, Foster City, USA) und MacLab D/A-Umwandler und Software (ADtnstruments, Castle Hill, Australia). Die erfindungsgemäßen Substanzen wurden getestet, indem sie in unterschiedlichen Konzentrationen der Badlösung zugefügt wurden. Die Effekte der Substanzen wurden als prozentuale Inhibition des Kv1.5-Kontrollstromes berechnet, der erhalten wurde, wenn der Lösung keine Substanz zugesetzt wurde. Die Daten wurden anschließend mit der Hill-Gleichung extrapoliert, um die Hemmkonzentrationen IC₅₀ für die jeweiligen Substanzen zu bestimmen.

Auf diese Weise wurden für die nachfolgend aufgeführten Verbindungen folgende IC₅₀-Werte bestimmt:

| Beispiel Nr. | IC50 [µM] |
|---|---|
| 1 | 4,7 |
| 2 | 0,7 |
| 3 | 1,4 |
| 4 | 0,2 |
| 5 | 10 |
| 6 | 1,0 |
| 7 | 1,1 |

Untersuchung der Refraktärzeit an normalen Ziegen und an Ziegen, mit remodellierten Vorhöfen

An chronisch instrumentierten Ziegen wurden die elektrophysiologischen Effekte der Verbindung des Beispiels 1, des IKᵣ-Kanalblockers Ibutilide und der Kombination von der Verbindung des Beispiels 1 und Ibutilide untersucht. Die Versuche wurden durchgeführt an normalen Vorhöfen von gesunden Ziegen, sowie an Vorhöfen, bei denen aufgrund eines 1 - 4 tägigen bestehenden atrialen Flimmerns ein sogenanntes elektrisches Remodelling vorlag. Dieses elektrische Remodelling, das die Situation am Patienten wiederspiegelt, wurde erreicht durch repetitive atriale Stimulationen wie publiziert von Wijffels et al (Atrial fibrillation begets atrial fibrillation. A study in awake chronical instrumented goats. Circulation 1995, 92, 1954-68).

Nach Infusion von 0,9% Kochsalzlösung über 30 min zur Kontrolle wurden die Substanzen lbutilide und die Verbindung des Beispiels 1 zum einen in Monotherapie und zum anderen in Kombination der beiden Wirkstoffe an 7 normalen Ziegen (d. h. ohne induziertes Vorhofflimmem) durch intravenöse Infusion verabreicht und die atriale effektive Refraktärzeit (AERP) bestimmt. Bei der Monotherapie wurde über die Zeitdauer von 60 Minuten 0.12 mg Ibutilide pro kg Körpergewicht verabreicht (0.002 mg pro kg pro Minute). In der Kombination wurden über die Dauer von 120 Minuten 6 mg der Verbindung des Beispiels 1 pro kg Körpergewicht verabreicht (3mg pro kg pro Stunde), wobei nach 60 Minuten zusätzlich 0.12 mg Ibutilide pro kg pro Stunde für die Dauer von 60 Minuten verabreicht wurden. Die Infusion der Verbindung des Beispiels 1 wurde insgesamt über 120 min durchgeführt. Kontrollmessungen der atrialen effektiven Refraktärzeit wurden einmal vor Gabe des Ibutilides (Kontrolle 1) und einmal vor Gabe der Verbindung des Beispiels 1 (Kontrolle 2) durchgeführt.

Die Ergebnisse der Messungen der atrialen effektiven Refraktärzeit (AERP) bei einer Zyklustänge von 400 ms in normalen (a) und remodellierten (b) Vorhöfen von 7 Ziegen sind in Tabelle 1 zusammengefasst. Ibutilide alleine verlängerte die AERP um 26 ms (n=7) und die Verbindung des Beispiels 1 um 44 ms (n=7). Diese Verlängerungen waren statistisch signifikant (p<0.001) gegenüber den Kontrollwerten 159 ms und 162 ms (jeweils vor Substanzgabe). Die Kombination beider Substanzen wurde getestet, indem wie oben beschrieben nach der Infusion der Verbindung des Beispiels 1 über eine Stunde zusätzlich Ibutilide verabreicht wurde. Es ergab sich nach der kombinierten Gabe eine Zunahme der AERP um 72 ms (n=7). Man erkennt, dass bei der kombinierten Gabe die Effekte additiv sind.

Das gleiche Infusionsschema wurde an denselben Tieren wiederholt, nachdem elektrische Remodellierung durch induziertes Vorhofflimmern von 1 bis 4 Tagen erzeugt wurde. Die Ergebnisse sind in Tabelle 1 zusammengefasst. Es ist zu erkennen, dass in diesen Tieren die AERP unter Kontrollbedingungen nur noch 93 ms bzw. 97 ms (n=7) betrug. Nach Gabe von Ibutilide betrug die Verlängerung des AERP 5 ms (n=7) und die von Verbindung des Beispiels 1 63 ms (n=7). Nach kombinierter Gabe wurde eine Zunahme des AERP um 94 ms (n=7) beobachtet.

**Tabelle 1: Mittelwert ± SD der effektiven atrialen Refraktärzeiten, gemessen an 7 Tieren.**

| | Kontrolle 1 (ms) | Ibutilide (ms) | Kontrolle 2 (ms) | Verbindung des Beispiels 1 (ms) | Verbindung des Beispiels 1 + Ibutilide (ms) |
|---|---|---|---|---|---|
| Nicht remodelliert | 159 ± 16 | 185 ± 23 | 162 ± 23 | 206 ± 26 | 234 ± 31 |
| | | | | | |
| Remodelliert | 93± 18 | 98 ± 20 | 97 ± 19 | 160 ± 16 | 191 ± 28 |

Ein analoges Infusionsschema wurde an 5 weiteren Tieren mit Dofetilide anstelle von Ibutilide durchgeführt. Nach Infusion von 0,9% Kochsalzlösung über 30 min zur Kontrolle wurden die Substanzen Dofetilide und die Verbindung des Beispiels 1 zum einen in Monotherapie und zum anderen in Kombination der beiden Wirkstoffe an 5 normalen Ziegen (d. h. ohne induziertes Vorhofflimmern) durch intravenöse Infusion verabreicht und die atriale effektive Refraktärzeit (AERP) bestimmt. Bei der Monotherapie wurde über die Zeitdauer von 60 Minuten 0.12 mg Dofetilide pro kg Körpergewicht verabreicht (0.002 mg pro kg pro Minute). In der Kombination wurden über die Dauer von 120 Minuten 6 mg der Verbindung des Beispiels 1 pro kg Körpergewicht verabreicht (3mg pro kg pro Stunde), wobei nach 60 Minuten zusätzlich 0.12 mg Dofetilide pro kg pro Stunde für die Dauer von 60 Minuten verabreicht wurden. Die Infusion der Verbindung des Beispiels 1 wurde insgesamt über 120 min durchgeführt. Kontrollmessungen der atrialen effektiven Refraktärzeit wurden einmal vor Gabe des Dofetilids (Kontrolle 1) und einmal vor Gabe der Verbindung des Beispiels 1 (Kontrolle 2) durchgeführt.

Die Ergebnisse der Messungen der atrialen effektiven Refraktärzeit (AERP) bei einer Zykluslänge von 400 ms in normalen (a) und remodellierten (b) Vorhöfen von 7 Ziegen sind in Tabelle 2 zusammengefasst. Dofetilide alleine verlängerte die AERP um 21 ms (n=5) und die Verbindung des Beispiels 1 um 51 ms (n=5). Diese Verlängerungen waren statistisch signifikant (p<0.001) gegenüber den Kontrollwerten 151 ms und 158 ms (jeweils vor Substanzgabe). Die Kombination beider Substanzen wurde getestet, indem wie oben beschrieben nach der Infusion der Verbindung des Beispiels 1 über eine Stunde zusätzlich Dofetilide verabreicht wurde. Es ergab sich nach der kombinierten Gabe eine Zunahme der AERP um 75 ms (n=5). Man erkennt, dass bei der kombinierten Gabe die Effekte additiv sind.

Das gleiche Infusionsschema wurde an denselben Tieren wiederholt, nachdem elektrische Remodellierung durch induziertes Vorhofflimmern von 1 bis 4 Tagen erzeugt wurde. Die Ergebnisse sind in Tabelle 2 zusammengefasst. Es ist zu erkennen, dass in diesen Tieren die AERP unter Kontrollbedingungen nur noch 94 ms bzw. 93 ms (n=5) betrug. Nach Gabe von Dofetilide betrug die Verlängerung des AERP 7 ms (n=5) und die von Verbindung des Beispiels 1 71 ms (n=5). Nach kombinierter Gabe wurde eine Zunahme des AERP um 91 ms (n=5) beobachtet.

**Tabelle 2: Mittelwert ± SD der effektiven atrialen Refraktärzeiten, gemessen an 5 Tieren.**

| | Kontrolle (ms) | Dofetilide 1 (ms) | Kontrolle 2 (ms) | Verbindung des Beispiels 1 (ms) | Verbindung des Beispiels 1 + Dofetilide (ms) |
|---|---|---|---|---|---|
| Nicht remodelliert | 151 ± 8 | 172 ± 11 | 158 ± 15 | 209 ± 29 | 234 ± 30 |
| | | | | | |
| Remodelliert | 94± 17 | 101 ± 19 | 93 ± 19 | 164 ± 19 | 184 ± 23 |

Aus diesen Versuchen ist somit folgendes zu erkennen. Der positive Effekt auf den AERP bei Verabreichung von Ibutilide oder Dofetilide ist bei remodelliertem Vorhof deutlich geringer als bei normalen Vorhof. Dieser Effekt ist bei der Verabreichung von der Verbindung des Beispiels 1 bei remodelliertem Vorhof deutlich größer als bei normalem Vorhof. Bei der kombinierten Gabe von Ibutilide und der Verbindung des Beispiels 1 ist der Effekt auf den AERP bei remodelliertem Vorhof deutlich größer als beim normalen Vorhof und auch deutlich größer als additive Effekte der beiden einzelnen Wirkstoffe, was die synergistische Wirkung der Kombination beweist.

### Untersuchung der Kardioversion an chronisch flimmemden Ziegen

Es wurden Versuche an zwei Ziegen durchgeführt, die sich in persistierendem Vorhofflimmem von mehr als 45 Tagen befanden. Nach Infusion von 0,9% Kochsalzlösung über 30 min zur Kontrolle, wurde einer Ziege die Verbindung des Beispiels 1 der Reihe nach (jeweils im Abstand von Tagen) in Mengen von jeweils 0.0 (Kontrolle), 0.1, 0.3, 1, 3 und 10 mg pro kg Körpergewicht und pro Stunde über eine Dauer von 90 Minuten infundiert, wobei nach 60 Minuten zusätzlich 2 mg Ibutilide pro Kilogramm Körpergewicht in einem Zeitintervall von 30 min verabreicht wurde.

Analog wurden einer zweiten Ziege nach 60 Minuten Verabreichung verschiedener Mengen der Verbindung des Beispiels 1 (jeweils 0.0 (Kontrolle), 0.1, 0.3, 1, 3 und 10 mg pro kg Körpergewicht und pro Stunde jeweils im Abstand von Tagen) zusätzlich 10 µg Dofetilide pro kg Körpergewicht in einem Zeitintervall von 30 Minuten anstelle von Ibutilide verabreicht.

Die Zeitpunkte der Kardioversion von persistierendem Vorhofflimmem (AF) durch die Gabe der Verbindung des Beispiels 1 mit Ibutilide bzw. der Verbindung des Beispiels 1 mit Dofetilide sind in den Tabellen 3 und 4 bei verschiedenen Dosierungen der Verbindung des Beispiels 1 (0.0, 0.1, 0.3, 1,3 und 10 mg pro kg Körpergewicht und pro Stunde) dargestellt.

**Tabelle 3: Auftreten der Kardioversion von persistierendem Vorhofflimmern (AF) nach jeweiliger Reinduktion des Vorhofflimmerns bei verschiedenen Dosierungen der Verbindung des Beispiels 1 und bei zusätzlicher Infusion von Ibutilide nach 60 Minuten (Zeitpunkte in Minuten gemessen ab Beginn der Infusion der Verbindung des Beispiels 1)**

| Dosierung der Verbindung des Beispiels 1 | | | | | |
|---|---|---|---|---|---|
| 0mg/kg/h | 0.1mg/kg/h | 0.3mg/kg/h | 1mg/kg/h | 3mg/kg/h | 10mg/kg/h |
| Zeitpunkte (Minuten) | | | | | |
| | | 82.00 | 71.00 | 65.55 | 42.00 |
| | | 88.00 | 71.30 | 68.10 | 50.00 |
| | | | 72.00 | 68.20 | 74.24 |
| | | | 74.00 | 69.49 | 74.30 |
| | | | 77.00 | 69.55 | 74.34 |
| | | | 79.00 | 70.50 | 74.42 |
| | | | 81.00 | 71.26 | 74.50 |
| | | | 81.10 | 71.43 | 74.58 |
| | | | 82.10 | 71.56 | 75.10 |
| | | | 84.00 | 72.09 | 76.55 |
| | | | 84.30 | 72.20 | 77.00 |
| | | | 84.50 | 72.36 | 77.30 |
| | | | 85.00 | 73.14 | 77.35 |
| | | | 85.30 | 74.00 | 77.40 |
| | | | | 74.20 | 78.10 |
| | | | | 74.29 | 78.20 |
| | | | | 74.39 | 78.30 |
| | | | | 75.00 | 78.35 |
| | | | | 75.10 | 78.48 |
| | | | | 75.40 | 79.10 |
| | | | | 75.43 | 79.20 |
| | | | | 75.48 | 79.30 |

**Tabelle 4: Auftreten der Kardioversion von persistierendem Vorhofflimmern (AF) nach jeweiliger Reinduktion des Vorhofflimmerns bei verschiedenen Dosierungen der Verbindung des Beispiels 1 und bei zusätzlicher Infusion von Dofetilide nach 60 Minuten (Zeitpunkte in Minuten gemessen ab Beginn der Infusion der Verbindung des Beispiels 1)**

| Dosierung der Verbindung des Beispiels 1 | | | | | |
|---|---|---|---|---|---|
| 0mg/kg/h | 0.1mg/kg/h | 0.3mg/kg/h | 1mg/kg/h | 3mg/kg/h | 10mg/kg/h |
| Zeitpunkte (Minuten) | | | | | |
| | | | | 65.50 | 83.00 |
| | | | | 66.10 | 83.30 |
| | | | | 70.57 | 84.30 |
| | | | | 73.45 | 84.37 |
| | | | | 73.55 | 85.22 |
| | | | | 74.30 | 85.34 |
| | | | | 75.20 | 85.47 |
| | | | | 75.50 | 85.50 |
| | | | | 76.40 | 85.58 |
| | | | | 79.40 | 86.14 |
| | | | | 80.40 | |
| | | | | 81.09 | |
| | | | | 81.30 | |

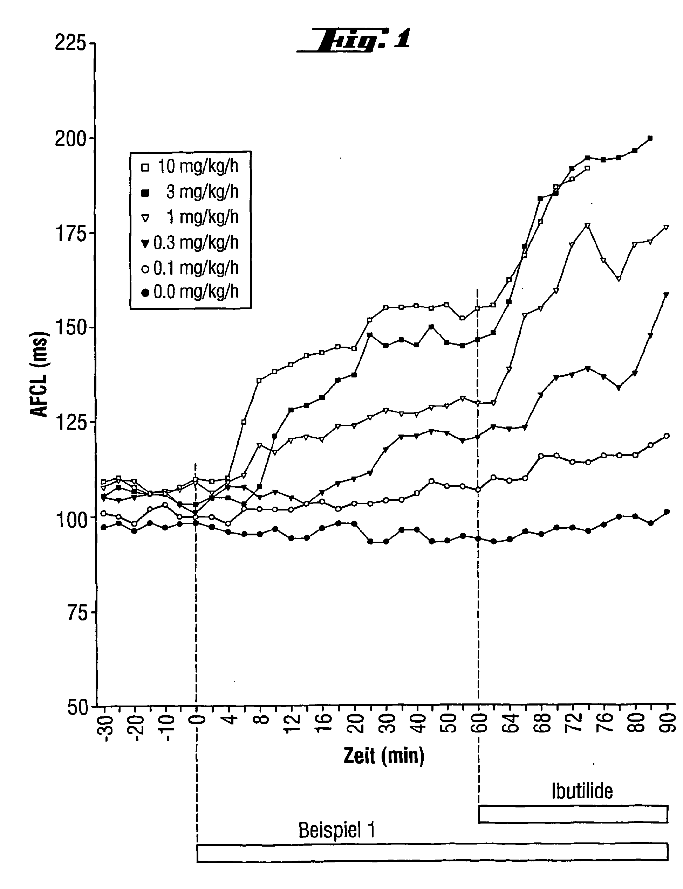

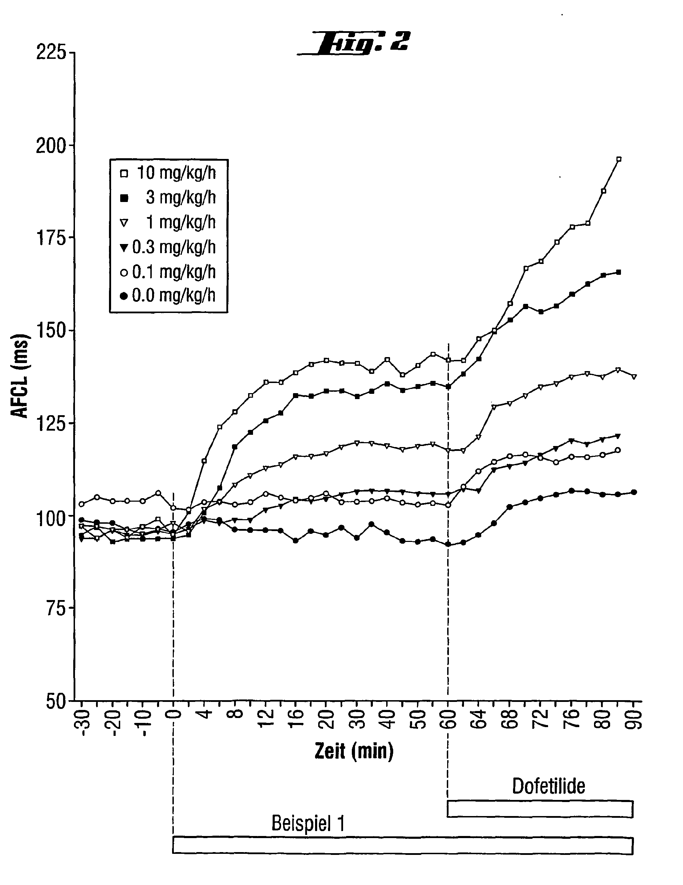

In Fig.1 und Fig.2 sind die Messungen der Zykluslänge des atrialen Flimmerns (AFCL) nach kombinierter Gabe von Ibutilide und der Verbindung des Beispiels 1 bzw. nach kombinierter Gabe von Dofetilide und der Verbindung des Beispiels 1 dargestellt. Die Messungen des AFCL und der Kardioversion erfolgten nebeneinander.

Die Tabellen 3 und 4 demonstrieren ebenso wie Fig.1 und 2, dass die Gabe von Kochsalzlösung, d.h. von 0.0 mg Verbindung des Beispiels 1 pro kg Körpergewicht und pro Stunde, keinen signifikanten Effekt in Bezug auf die AFCL und die Kardioversion verursacht.

Ibutilide allein führt nur zu einer sehr geringen Zunahme der AFCL von 94 ms auf 101 ms (Fig.1, 0.0 mg/kg/h, Gabe von Ibutilide nach 60 Minuten). Nach Behandlung mit 3 mg der Verbindung des Beispiels 1 pro kg Körpergewicht pro Stunde verlängert die gleiche Dosis von Ibutilide (2 mg pro kg Körpergewicht pro 30 Minuten) die AFCL um 50 ms (von 147 ms auf 197 ms). In der Monotherapie mit der Verbindung des Beispiels 1 war es erst durch Gabe von 10 mg der Verbindung des Beispiels 1 pro kg Körpergewicht pro Stunde möglich, eine Kardioversion herbeizuführen (Tabelle 3). Im Gegensatz hierzu führten bei der Kombination mit Ibutilide bereits die niedrigeren Dosen von 0.3, 1, und 3 mg der Verbindung des Beispiels 1 pro kg Körpergwicht pro Stunde zur Terminierung des Vorhofflimmerns (Tabellen 3).

Ähnliche Ergebnisse wurden mit Dofetilide erzielt. Fig. 2 und Tabelle 4 zeigen, dass nach Infusion von 10 µg Dofetilide pro kg Körpergewicht über 30 min ohne die Verbindung des Beispiels 1 die AFCL nur um 14 ms (von 92 ms auf 106 ms) ansteigt und eine Kardioversion nicht eintritt. Die Infusion von der Verbindung des Beispiels 1 in den verschiedenen Dosen (0.1, 0.3, 1, 3 oder 10) allein führt zu einer dosisabhängigen Zunahme der AFCL, aber nicht zur Kardioversion. Erst unter der Kombination von der Verbindung des Beispiels 1 und Dofetilide bewirken die Dosen von 3 und 10 mg der Verbindung des Beispiels 1 pro kg Körpergewicht und pro Stunde eine Terminierung des Vorhofflimmerns (Tabelle 4).

Aus diesen Versuchen ist also erkennbar, dass eine Kombination von der Verbindung des Beispiels 1 mit Ibutilide oder Dofetilide bei niedrigen Dosen von der Verbindung des Beispiels 1 und sehr niedrigen Dosen Ibutilide oder Dofetilide zu einer Kardioversion und damit zu einer Terminierung des Flimmerns führt.

In weiteren Versuchen wurde die Effizienz der Kardioversion bei verschiedenen Dosierungen der Verbindung des Beispiels 1 und bei zusätzlicher Infusion von Ibutilide (2 mg/kg) bzw. Dofetilide (10 µg/kg) an mehreren Ziegen getestet (Tabelle 5).

Es wurden Versuche an bis zu acht Ziegen durchgeführt, die sich in persistierendem Vorhofflimmern befanden. Nach Infusion von 0,9% Kochsalzlösung über 30 min zur Kontrolle wurden bis zu acht Ziegen die Verbindung des Beispiels 1 (jeweils im Abstand von Tagen) in Mengen von jeweils 0.0 (Kontrolle), 0.1, 0.3, 1, 3 und 10 mg pro kg Körpergewicht und pro Stunde über eine Dauer von 90 Minuten infundiert, wobei nach 60 Minuten zusätzlich 2 mg Ibutilide pro Kilogramm Körpergewicht in einem Zeitintervall von 30 min verabreicht wurde.

Analog wurden an bis zu acht Ziegen nach 60 Minuten Verabreichung verschiedener Mengen der Verbindung des Beispiels 1 (jeweils 0.0 (Kontrolle), 0.1, 0.3, 1,3 und 10 mg pro kg Körpergewicht und pro Stunde jeweils im Abstand von Tagen) zusätzlich 10 µg Dofetilide pro kg Körpergewicht in einem Zeitintervall von 30 Minuten anstelle von Ibutilide verabreicht.

Die Effizienz der Kardioversion von persistierendem Vorhofflimmern (AF) durch die Gabe der Verbindung des Beispiels 1 mit ibutilide bzw. der Verbindung des Beispiels 1 mit Dofetilide sind in der Tabelle 5 bei verschiedenen Dosierungen der Verbindung des Beispiels 1 (0.0, 0.1, 0.3, 1, 3 und 10 mg pro kg Körpergewicht und pro Stunde) dargestellt. Angegeben sind die Anzahl der Ziegen, bei denen Kardioversion auftrat, im Vergleich zu der Anzahl der Ziegen, mit denen der Versuch durchgeführt wurde, und daraus errechnet die Effizienz der Kardioversion in Prozent. Jede Terminierung des persistenten Vorhofflimmerns während einer jeweiligen Infusionsperiode wurde als eine erfolgreiche Kardioversion angesehen. Während einer einstündigen Infusion führte beispielsweise die Gabe der Verbindung des Beispiels 1 bei einer Dosierung von 3 mg/kg/h bei 3 von 8 Ziegen zur Terminierung des AF. Bei Gabe des Beispiels 1 (3 mg/kg/h) in Kombination mit Ibutilide war bei 6 von 7 Ziegen AF terminiert. Wurde die Behandlung mit einer Dosis von 10 mg/kg/h der Verbindung des Beispiels 1 durchgeführt, so konnte das persistente Vorhofflimmern bei zusätzlicher Infusion von Ibutilide oder Dofetilide in jeder Ziege terminiert werden.

**Tabelle 5: Effizienz der Kardioversion in Prozent bei Ziegen mit persistierendem Vorhofflimmern (AF) bei verschiedenen Dosierungen der Verbindung des Beispiels 1 und bei zusätzlicher Infusion von Ibutilide bzw. Dofetilide. In Klammern angegeben ist jeweils das Verhältnis Ziegen, bei denen Kardioversion auftrat, zu Ziegen, die bei dem jeweiligen Versuch eingesetzt wurden.**

| Verbindung des Beispiels 1 (mg/kg) | Effizienz der Cardioversion | | |
|---|---|---|---|
| | Monotherapie | + Ibutilide (2mg/kg) | + Dofetilide (10µg/kg) |
| 0 | 0% | 29% | 25% |
| | (0/8) | (2/7) | (1/4) |
| 0,1 | 17% | 33% | 33% |
| | (1/6) | (1/3) | (1/3) |
| 0,3 | 17% | 50% | 0% |
| | (1/6) | (2/4) | (0/2) |
| 1 | 13% | 75% | 50% |
| | (1/8) | (3/4) | (2/4) |
| 3 | 38% | 86% | 75% |
| | (3/8) | (6/7) | (3/4) |
| 10 | 63% | 100% | 100% |
| | (5/8) | (5/5) | (4/4) |

In den Zeichnungen wurden folgende Beschriftungen und Kennzeichnungen vorgenommen:
Fig.1: Messung der Zykluslänge des atrialen Flimmerns (AFCL) nach kombinierter Gabe von Ibutilide und der Verbindung des Beispiels 1
   Y-Achse: AFCL in ms
   X-Achse: Zeit in Minuten
   Die Verbindung des Beispiels 1 wurde in folgenden Mengen infundiert:
   10 mg/kg/h
   3 mg/kg/h
   1 mg/kg/h
   0.3 mg/kg/h
   0.1 mg/kg/h
   0.0 mg/kg/h (Kontrolle)
Fig.2: Messung der Zykluslänge des atrialen Flimmerns (AFCL) nach kombinierter Gabe von Dofetilide und der Verbindung des Beispiels 1
   Y-Achse: AFCL in ms
   X-Achse: Zeit in Minuten
   Die Verbindung des Beispiels 1 wurde in folgenden Mengen infundiert:
   10 mg/kg/h
   3 mg/kg/h
   1 mg/kg/h
   0.3 mg/kg/h
   0.1 mg/kg/h
   0.0 mg/kg/h (Kontrolle)

## Patentansprüche

1. Kombination aus 2'-{[2-(4-Methoxy-phenyl)-acetylamino]-methyl}-biphenyl-2-carbonsäure-(2-pyridin-3-yl-ethyl)-amid und aus einem oder mehreren IKr-Kanalblockern ausgewählt aus der Gruppe Dofetilide und Ibutilide, und/oder ihren physiologisch verträglichen Salzen.

2. Kombination nach Anspruch 1, wobei die Komponenten auch in Form ihrer physiologisch verträglichen Salze vorliegen können, enthaltend:
2'-{[2-(4-Methoxy-phenyl)-acetylamino]-methyl}-biphenyl-2-carbonsäure-(2-pyridin-3-yl-ethyl)-amid und lbutilide, oder
2'-{[2-(4-Methoxy-phenyl)-acetylamino]-methyl}-biphenyl-2-carbonsäure-(2-pyridin-3-yl-ethyl)-amid und Dofetilide.

3. Pharmazeutische Zubereitung, enthaltend eine Kombination nach einem der Ansprüche 1 oder 2 und/oder physiologisch verträglicher Salze davon als Wirkstoff, zusammen mit pharmazeutisch annehmbaren Träger- und Zusatzstoffen und gegebenenfalls noch einem oder mehreren anderen pharmakologischen Wirkstoffen.

4. Erzeugnis, enthaltend eine Kombination nach einem der Ansprüche 1 oder 2 und/oder physiologisch verträglicher Salze davon zur gleichzeitigen, getrennten oder zeitlich abgestuften Anwendung zur Therapie oder Prophylaxe von atrialer Fibrillation oder atrialem Flattern.

5. Verwendung einer Kombination nach einem der Ansprüche 1 oder 2 und/oder physiologisch verträglicher Salze davon, zur Herstellung eines Medikamentes zur Therapie oder Prophylaxe von atrialer Fibrillation oder atrialem Flattern.

6. Kombination nach einem der Ansprüche 1 oder 2 zur Therapie oder Prophylaxe von atrialer Fibrillation oder atrialem Flattern.

## Claims

1. A combination of 2'-{[2-(4-methoxyphenyl)acetylamino]methyl}biphenyl-2-carboxylic acid (2-pyridin-3-ylethyl)amide and of one or more IKr channel blockers selected from the group consisting of dofetilide and ibutilide, and/or their physiologically tolerable salts.

2. The combination as claimed in claim 1, it also being possible for the components to be present in the form of their physiologically tolerable salts, comprising:
2'-{[2-(4-methoxyphenyl)acetylamino]methyl}biphenyl-2-carboxylic acid (2-pyridin-3-ylethyl)amide and ibutilide, or
2'-{[2-(4-methoxyphenyl)acetylamino]methyl}biphenyl-2-carboxylic acid (2-pyridin-3-ylethyl)amide and dofetilide.

3. A pharmaceutical preparation comprising a combination as claimed in either of claims 1 and 2 and/or physiologically tolerable salts thereof as active compound, together with pharmaceutically acceptable vehicles and additives and, if appropriate, additionally one or more other pharmacological active compounds.

4. A product comprising a combination as claimed in either of claims 1 and 2 and/or physiologically tolerable salts thereof for simultaneous, separate or sequential use for the therapy or prophylaxis of atrial fibrillation or atrial flutters.

5. The use of a combination as claimed in either of claims 1 and 2 and/or of physiologically tolerable salts thereof in the manufacture of a medicament for the therapy or prophylaxis of atrial fibrillation or atrial flutters.

6. The combination as claimed in either of claims 1 and 2 for the therapy or prophylaxis of atrial fibrillation or atrial flutters.

## Revendications

1. Combinaison de (2-pyridin-3-yléthyl)-amide de l'acide 2'-{[2-(4-méthoxyphényl)-acétylamino]-méthyl}-biphényl-2-carboxylique et d'un ou de plusieurs bloquants du canal IKr choisi(s) dans le groupe de Dofétilide et d'Ibutilide, et/ou leurs sels physiologiquement acceptables.

2. Combinaison selon la revendication 1, où les composants peuvent également se trouver sous forme de leurs sels physiologiquement acceptables, contenant :
- du (2-pyridin-3-yléthyl)-amide de l'acide 2'-{[2-(4-méthoxyphényl)-acétylamino]-méthyl}-biphényl-2-carboxylique et de l'Ibutilide, ou
- du (2-pyridin-3-yléthyl)-amide de l'acide 2'-{[2-(4-méthoxyphényl)-acétylamino]-méthyl}-biphényl-2-carboxylique et du Dofétilide.

3. Préparation pharmaceutique, contenant une combinaison selon l'une quelconque des revendications 1 à 2 et/ou des sels physiologiquement acceptables de ses composants comme substance active avec des supports et des additifs pharmaceutiquement acceptables et le cas échéant encore une ou plusieurs autres substances actives pharmacologiques.

4. Produit, contenant une combinaison selon l'une quelconque des revendications 1 ou 2 et/ou des sels physiologiquement acceptables de ses composants destiné à une utilisation simultanée, séparée ou échelonnée dans le temps pour la thérapie ou la prophylaxie de la fibrillation atriale ou de l'arythmie atriale.

5. Utilisation d'une combinaison selon quelconque des revendications 1 à 2 et/ou des sels physiologiquement acceptables de ses composants pour la préparation d'un médicament destiné à la thérapie ou la prophylaxie de la fibrillation atriale ou de l'arythmie atriale.

6. Combinaison selon l'une quelconque des revendications 1 ou 2 pour la thérapie ou la prophylaxie de la fibrillation atriale ou de l'arythmie atriale.
